# EUROPEAN PATENT APPLICATION

(11) **EP 1 767 650 A1**
(43) Date of publication of application: **28.03.2007**
(21) Application number: 05753409.1
(22) Date of filing: 21.06.2005
(51) Int. Cl.: C12Q 1/48, C12Q 1/54, C12N 15/54, C12N 9/10, G01N 33/15, G01N 33/50, A61K 38/00, A61K 45/00, A61K 48/00, A61P 3/10

(54) **METHOD OF SCREENING COMPOUND DIRECTLY ACTIVATING GLYCOGEN SYNTHASE**

(30) Priority: 25.06.2004 JP 2004188711
(71) Applicant: BANYU PHARMACEUTICAL CO., LTD., Tokyo 102-8667 (JP)
(72) Inventor: KADOTANI, Akito, Banyu Pharmaceutical Co.,Ltd, Tsukuba-shi, Ibaraki 3002611 (JP); NAGATA, Yasufumi, BANYU PHARM. CO., LTD., Tsukuba-shi, Ibaraki 3002611 (JP); NAKAMURA, Takao, BANYU PHARM CO., LTD, Tsukuba-shi, Ibaraki 3002611 (JP); SUZUKI, Maho, BANYU PHARM. CO., LTD, Tsukuba-shi, Ibaraki 3002611 (JP); EIKI, Jun-ichi, BANYU PHARM.CO., LTD, Tsukuba-shi, Ibaraki 3002611 (JP)
(74) Representative: Rollins, Anthony John
(86) International application number: PCT/JP2005/011353
(87) International publication number: WO 2006/001275

(57) **Abstract**

The present invention provides a therapeutic agent for diabetes comprising a compound directly activating glycogen synthase as an active ingredient. The present invention further provides a method for screening compounds directly activating glycogen synthase.

## Description

### Technical Field

The present invention relates to compounds directly activating glycogen synthase.

### Background Art

It is known that glycogen synthase in human exists in two types of genes and enzymes: a muscle type (glycogen synthase 1; 737 amino acids, 83.6 kDa) and a liver type (glycogen synthase 2; 703 amino acids, 80.9 kDa), and functions as a rate-determining enzyme in glycogen synthesis (Non-patent document 1).

In the liver, excess sugar after meal is stored as glycogen by the action of glycogen synthase, whereas while fasting, glycogenolysis occurs by glycogen phosphorylase, and the generated sugar is released from the liver. In this way, glycogen synthase in combination with glycogen phosphorylase is thought to participate in blood sugar regulation.

Glycogen synthase activity is regulated by phosphorylation and glucose-6-phosphate (G-6-P). Glycogen synthase has seven serine phosphorylation sites (in the order of site 2, 2a, 3a, 3b, 3c, 4, and 5 from the N terminal side). When these sites are phosphorylated by various kinases, glycogen synthase is changed into an inactive form, whereas when dephosphorylated by protein phosphatase 1G, the inactive form is changed into an active form. Further, glycogen synthase in both phosphorylated and dephosphorylated forms is strongly activated up to a certain activity level by an endogenous allosteric activation factor, G-6-P (Non-patent document 1).

It is known that since persons with mutations leading to a reduced activity form of glycogen synthase cannot store glycogen, sugar release from the liver is enhanced, resulting in postprandial hyperglycemia (Non-patent documents 2 and 3). It is also known that liver glycogen is reduced in patients with type 1 and type 2 diabetes who are in hyperglycemic states (Non-patent documents 4 to 7). From these clinical findings, the relationship between reduction in glycogen synthase activity and hyperglycemia is suggested, and the development of therapeutic agents for diabetes is in progress based on the activation of glycogen synthase.

Non-patent document 1: Juan C. Ferrer et al., FEBS Letters, 546, 127-132 (2003)
Non-patent document 2: M. Orho et al., J. Clin. Invest., 102, 507-515 (1998)
Non-patent document 3: B. E. Bachrach et al., J. Pediatr., 140, 781-783 (2002)
Non-patent document 4: J. H. Hwang et al., J. Clin. Invest., 95, 783-787 (1995)
Non-patent document 5: G Velho et al., J. Clin. Invest., 98, 1755-1761 (1996)
Non-patent document 6: M. G Bischof et al., Diabetes, 51, 49-54 (2002)
Non-patent document 7: I. Magnusson et al., J. Clin. Invest., 90, 1323-1327 (1992)

### Disclosure of the Invention

### Problems to be Solved by the Invention

All therapeutic agents for diabetes being developed among those based on the activation of glycogen synthase are based on an action to indirectly increase glycogen synthase activity. That is, these agents use as an active ingredient an inhibitor of glycogen synthase kinase 3 that changes glycogen synthase into an inactive form by phosphorylation.

However, in addition to glycogen synthase kinase 3, there are many enzymes (kinases) *in vivo* that change glycogen synthase into an inactive form by phosphorylation, and the inhibitor of glycogen synthase kinase 3 cannot inhibit all of these kinases. In other words, even when glycogen synthase kinase 3 is inhibited, glycogen synthase is phosphorylated by other kinases to result in inactivation, and therefore, there is a possibility that hyperglycemia cannot ultimately be controlled.

Hence, the object of the present invention is to provide a therapeutic agent for diabetes that enhances glycogen synthase activity without being affected by various kinases.

### Means for Solving the Problems

In order to accomplish the above object, the present invention provides a therapeutic agent for diabetes comprising a compound directly activating glycogen synthase as an active ingredient. By directly activating glycogen synthase, the glycogen synthase becomes unaffected by kinases present *in vivo* that inactivate glycogen synthase and thus control of hyperglycemia becomes possible. Further, the present inventors have found that the compound directly activating glycogen synthase is different from other therapeutic agents for diabetes and has a possibility to lower the risk of hypoglycemia. Furthermore, the present inventors have also found a possibility that the compound directly activating glycogen synthase improves obesity and can be applied to both type 1 and type 2 diabetes.

In order to search for compounds directly activating glycogen synthase, the present inventors developed a method for screening the compounds. That is, the present invention is a method for screening the compounds directly activating glycogen synthase and provides the method comprising the steps of (a) allowing glycogen synthase and a candidate compound to contact each other to assay glycogen synthase activity under the condition that glucose-6-phosphatase, hexokinase, glycogen phosphorylase, glycogen synthase kinase 3, and protein phosphatase 1G are not present and (b) selecting a candidate compound having an activity assayed in the step (a) higher than that assayed in the same manner as in the step (a) except that the candidate compound is not used. Since factors that are involved in the regulation of glycogen synthase activity have been excluded from the screening system, compounds indirectly activating glycogen synthase are not supposed to be selected in this screening method.

As another method for screening the compounds directly activating glycogen synthase, the present invention further provides a screening method comprising the steps of (a) allowing glycogen synthase and a candidate compound to contact each other to assay glycogen synthase activity, (b) measuring the binding constant between glycogen synthase and the candidate compound, and (c) selecting a candidate compound showing an activity in the step (a) higher than that assayed in the same manner as in the step (a) except that the candidate compound is not used and having a binding constant equal to or larger than a predetermined value. Since only compounds that bind to glycogen synthase can be selected owing to the binding experiment, only compounds directly activating glycogen synthase can be selected in this screening method.

As still another method for screening the compounds directly activating glycogen synthase, the present invention further provides a screening method comprising the steps of (a) allowing glycogen synthase and a candidate compound to contact each other to assay glycogen synthase activity, (b) assaying an activity-modulating effect of the candidate compound on at least one enzyme selected from the group consisting of glucose-6-phosphatase, hexokinase, glycogen phosphorylase, glycogen synthase kinase 3, and protein phosphatase 1G, and (c) selecting a compound showing an activity in the step (a) higher than that assayed in the same manner as in the step (a) except that the candidate compound is not used and not showing any activity-modulating effect in the step (b). Since compounds that influence on factors involved in the regulation of glycogen synthase activity are excluded in the present screening method, compounds indirectly activating glycogen synthase are not supposed to be selected.

The glycogen synthase in the above screening method is preferably glycogen synthase 2. This is because, since the control of glycogen storage and sugar release in the liver is important in diabetes, it is preferred to perform screening focused on glycogen synthase 2 that is liver-type glycogen synthase from the viewpoint of specificity.

The present invention also provides an isolated nucleic acid comprising the nucleotide sequence according to SEQ ID NO:11, a vector inserted with the nucleic acid, and the vector for gene therapy. The present invention further provides an isolated protein comprising the amino acid sequence according to SEQ ID NO:12 and a therapeutic agent for diabetes comprising the protein as an active ingredient.

The nucleic acid comprising the nucleotide sequence according to SEQ ID NO:11 encodes a variant of human glycogen synthase 2 (protein comprising the amino acid sequence according to SEQ ID NO:12). In this variant, among 7 serine phosphorylation sites that glycogen synthase 2 has, six serine residues at sites 2, 3a, 3b, 3c, 4, and 5 are substituted by alanine residues. This variant lacking the phosphorylation sites is not converted to an inactive form by being phosphorylated, stays in an active form at all times, and is always able to carry out glycogen synthesis without being affected by kinases present *in vivo*. Accordingly, the nucleic acid comprising the nucleotide sequence according to SEQ ID NO:11 can be applied to gene therapy of diabetes. Further, the protein comprising the amino acid sequence according to SEQ ID NO:12 can be used for a therapeutic agent of diabetes.

The present invention also provides an isolated nucleic acid comprising the nucleotide sequence according to SEQ ID NO:13, a vector inserted with the nucleic acid, and the vector for gene therapy. The present invention further provides an isolated protein comprising the amino acid sequence according to SEQ ID NO:14 and a therapeutic agent for diabetes comprising the protein as an active ingredient.

The nucleic acid comprising the nucleotide sequence according to SEQ ID NO:13 encodes a variant of human glycogen synthase 2 (protein comprising the amino acid sequence according to SEQ ID NO:14). In this variant, all serine residues of the 7 serine phosphorylation sites that glycogen synthase 2 has (sites 2, 2a, 3a, 3b, 3c, 4, and 5) are substituted by alanine residues. This variant lacking the phosphorylation sites is not converted to an inactive form by being phosphorylated, stays in an active form at all times, and is always able to carry out glycogen synthesis without being affected by kinases present *in vivo*. Accordingly, the nucleic acid comprising the nucleotide sequence according to SEQ ID NO:13 can be applied to gene therapy of diabetes. Further, the protein comprising the amino acid sequence according to SEQ ID NO:14 can be used for a therapeutic agent of diabetes.

### Effect of the Invention

According to the present invention, a therapeutic agent for diabetes that enhances glycogen synthase activity without being affected by various kinases can be provided.

### Brief Description of the Drawings

Figure 1 is a schematic diagram of one example of methods for assaying glycogen synthase activity;
Figure 2 is a graph showing the amounts of glucose production by a control (LacZ) and variants of glycogen synthase 2 (AS-5A and AA-5A) at various multiplicities of infection; and
Figure 3 is a graph showing results of oral glucose tolerance test for a mouse with high expression of a variant of glycogen synthase 2 (AS-5A).

### Best Modes for Carrying Out the Invention

Hereinafter, preferable embodiments of the present invention are explained.

### (Method for screening compounds directly activating glycogen synthase)

The first method for screening compounds directly activating glycogen synthase of the present invention comprises the steps of (a) allowing glycogen synthase and a candidate compound to contact each other to assay glycogen synthase activity under the condition that glucose-6-phosphatase, hexokinase, glycogen phosphorylase, glycogen synthase kinase 3, and protein phosphatase 1G are not present and (b) selecting a compound having an activity assayed in the step (a) higher than that assayed in the same manner as in the step (a) except that the candidate compound is not used.

Many factors that exert an influence on glycogen synthase are present in cells. For example, glycogen synthase kinase 3 converts glycogen synthase to an inactive form by phosphorylation. Accordingly, when glycogen synthase activity is assayed in cells and the activity is enhanced by the presence of a candidate compound, it is not possible to distinguish whether the candidate compound directly activates glycogen synthase or a factor that suppresses glycogen synthase activity is inhibited. Therefore in the screening method of the present invention, glycogen synthase activity is assayed under the condition that factors involved in the regulation of glycogen synthase activity (glucose-6-phosphatase, hexokinase, glycogen phosphorylase, glycogen synthase kinase 3, and protein phosphatase 1G) are not present. By assaying the activity in this way, only candidate compounds that directly act on glycogen synthase to enhance the activity can be selected.

The method for assaying glycogen synthase activity under the condition that the factors involved in the regulation of glycogen synthase activity are not present specifically includes the following methods.

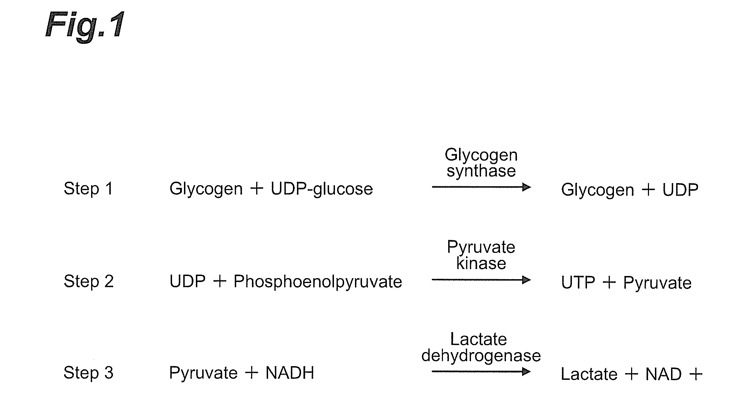

The generation of UDP (uridine 5'-diphosphate) by glycogen synthase is coupled with pyruvate kinase and lactate dehydrogenase, and decrease in NADH (reduced form of nicotinamide adenine dinucleotide) is assayed, whereby glycogen synthase activity can be assayed. The outline of this assay method is shown in Figure 1. First, from glycogen and UDP-glucose, glycogen having one additional glucose and UDP are produced by glycogen synthase as the first reaction. Next, from UDP and phosphoenolpyruvate, UTP and pyruvate are produced by pyruvate kinase. Finally, from pyruvate and NADH, lactate and NAD+ (oxidized form of nicotinamide adenine dinucleotide) are produced by lactate dehydrogenase. That is, as the reaction of glycogen synthase proceeds, NADH is consumed, and therefore the decreased amount of NADH reflects glycogen synthase activity. The determination of NADH can be carried out by measuring the absorbance at 340 nm or the fluorescence intensity (excitation wavelength: 340 nm, detection wavelength: 460 nm).

More specifically, a buffer (Tris-HCl buffer, etc.) in which enzymes, substrates, co-factors (pyruvate kinase, lactate dehydrogenase, glycogen, phosphoenolpyruvate, NADH, MgCl₂, etc.), and the like except for glycogen synthase and UDP-glucose are added is prepared. To this buffer, a candidate compound, glycogen synthase, and UDP-glucose are added to initiate enzyme reactions. After carrying out the enzyme reactions for a predetermined time at a predetermined temperature (for example, 30°C for 60 min), glycogen synthase activity can be assayed by measuring the absorbance (340 nm) or the fluorescence intensity (excitation wavelength: 340 nm, detection wavelength: 460 nm) of the reaction mixture to determine NADH. One skilled in the art can appropriately adjust the reaction conditions (kinds and amounts of additives, reaction temperature, reaction time, etc.).

When glycogen synthase activity in the presence of an added candidate compound is higher than that in the absence of the added candidate compound, the candidate compound can be judged to be a compound directly activating glycogen synthase.

Further, other methods for measuring glycogen synthase activity under the condition that factors involved in the regulation of glycogen synthase activity are not present include, for example, a method described below. From glycogen and UDP-[¹⁴C]glucose, [¹⁴C]glycogen and UDP are produced by glycogen synthase, and glycogen is recovered as pellets by ethanol precipitation, followed by determination of radioactivity of the pellets with a scintillation counter or the like. The radioactivity reflects glycogen synthase activity.

Next, the second method for screening compounds directly activating glycogen synthase of the present invention is explained. The second screening method comprises the steps of (a) allowing glycogen synthase and a candidate compound to contact each other to assay glycogen synthase activity, (b) measuring the binding constant between glycogen synthase and the candidate compound, and (c) selecting a candidate compound showing an activity in the step (a) higher than that assayed in the same manner as in the step (a) except that the candidate compound is not used and having a binding constant equal to or larger than a predetermined value.

In the present screening method, since only compounds capable of binding to glycogen synthase are selected by performing binding experiments, it is possible to select only compounds that directly act on glycogen synthase to enhance the activity.

In the present screening method, the method for assaying glycogen synthase activity is not particularly limited, and the assay may be carried out either under the condition that factors that suppress glycogen synthase activity are present (system using hepatocytes and the like) or under the condition that factors that suppress glycogen synthase activity are not present as described above.

As an assay system using hepatocytes and the like, a method in which glycogen synthase activity is assayed with cell extracts using incorporation of UDP-[¹⁴C]-glucose into glycogen as the index is listed. Here, the cell extracts may be prepared from any one of primary hepatocytes, cultured cells, and transgenic cultured cells. Further, as another assay system, a method in which, with the use of primary hepatocytes, cultured cells, or transgenic cultured cells, glycogen synthase activity is assayed by adding [¹⁴C]-glucose in a culture medium, allowing it to be incorporated into cells, and using its incorporation into glycogen by intracellular glycogen synthase as the index is listed.

For binding experiment to determine the binding constant between glycogen synthase and a candidate compound, a method known to one skilled in the art can be used. When the binding constant (Ka) is equal to or larger than a predetermined value, it can be inferred that glycogen synthase and the candidate compound can bind to each other. Here, the predetermined value depends on the assay condition and the like, but generally is a value in the range of 2x10³ M⁻¹ to 1x10⁶ M⁻¹, preferably 1x10⁴ M⁻¹.

A specific method of the binding experiment is, for example, as follows. Glycogen synthase linked with an affinity tag such as His-tag is immobilized on a plate and the like coated with a resin that binds to the affinity tag. Subsequently, radio-labeled glucose-6-phosphate and a candidate compound are added to the system to allow these to compete for the glycogen synthase. After removing unadsorbed labeled compound and unadsorbed compounds by washing with an appropriate buffer, the radioactivity of the system is assayed.

When glycogen synthase activity in the presence of an added candidate compound is higher than that in the absence of the added candidate compound and when the binding constant between glycogen synthase and the candidate compound is equal to or larger than a predetermined value, the candidate compound can be judged to be a compound directly activating glycogen synthase.

Next, the third method for screening compounds directly activating glycogen synthase of the present invention is explained. The third screening method comprises the steps of (a) allowing glycogen synthase and a candidate compound to contact each other to assay glycogen synthase activity, (b) assaying an activity-modulating effect of the candidate compound on at least one enzyme selected from the group consisting of glucose-6-phosphatase, hexokinase, glycogen phosphorylase, glycogen synthase kinase 3, and protein phosphatase 1 G, and (c) selecting a compound showing an activity in the step (a) higher than that assayed in the same manner as in the step (a) except that the candidate compound is not used and not showing any activity-modulating effect in the step (b).

Since the activity-modulating effect of a candidate compound on factors involved in the regulation of glycogen synthase activity is assayed and a candidate compound that shows such an activity-modulating effect is excluded in the present screening method, it is possible to select only compounds that directly act on glycogen synthase to enhance the activity.

In the present screening method, the method for assaying glycogen synthase activity is not particularly limited, and the assay may be carried out either under the condition that the factors involved in the regulation of glycogen synthase activity are present or under the condition that the factors involved in the regulation of glycogen synthase activity are not present.

As the method for assaying the activity-modulating effect of a candidate compound on the factors involved in the regulation of glycogen synthase activity, a method known to one skilled in the art can be used. When the activity-modulating effect is assayed and when the inhibition rate or the activation rate is equal to or lower than a predetermined value, the candidate compound can be considered not to exhibit any activity-modulating effect (inhibition effect or activation effect) on the factors involved in the regulation of glycogen synthase activity.

Alternatively, when the activation effect of a candidate compound on glycogen synthase shows 100-fold higher selectivity than the activity-modulating effect of the candidate compound on the factors involved in the regulation of glycogen synthase activity, the candidate compound can be considered a candidate compound that does not exhibit any activity-modulating effect on the factors involved in the regulation of glycogen synthase activity. In other words, when EC₅₀ (50% effective concentration) of the candidate compound for glycogen synthase is at least 100-fold lower than EC₅₀ or IC₅₀ (50% inhibitory concentration) of the candidate compound for the factors involved in the regulation of glycogen synthase activity, the candidate compound can be considered a candidate compound that does not exhibit any activity-modulating effect on the factors involved in the regulation of glycogen synthase activity.

The assay of the activity-modulating effect of a candidate compound on the factors involved in the regulation of glycogen synthase activity is possible by measuring changes in activity when a candidate compound is added to a system for determining the activity of a factor of interest (specific examples are shown below).

The activity of glucose-6-phosphatase can be determined, for example, by the following method. Glucose-6-phosphate is converted to glucose by glucose-6-phosphatase. Further, glucose is converted to glucose-6-phosphate by hexokinase, ADP generated from ATP at that time is coupled to the reactions of pyruvate kinase and lactate dehydrogenase, and decreased NADH is assayed.

The activity of hexokinase can be determined, for example, by the following method. Glucose-6-phosphate is produced from glucose and ATP by hexokinase. The product glucose-6-phosphate is coupled to the reaction of glucose-6-phosphate dehydrogenase, and generated NADH is assayed.

The activity of glycogen phosphorylase can be assayed, for example, by the following method. Glucose-1-phosphate is produced from glycogen and inorganic phosphate by glycogen phosphorylase. The product glucose-1-phosphate is converted to glucose-6-phosphate by phosphoglucomutase, glucose-6-phosphate is further converted to 6-phosphogluconate by glucose-6-phosphate dehydrogenase, and NADH generated in this reaction is assayed.

The activity of glycogen synthase kinase 3 can be assayed, for example, by the following method. With glycogen synthase as the substrate, [γ-³²P]ATP, MgCl₂, and glycogen synthase kinase 3 are incubated. After the reaction, protein is adsorbed onto a filter, washed with an appropriate buffer, and the radioactivity on the filter is assayed with a scintillation counter.

The activity of protein phosphatase 1G can be assayed, for example, by the following method. With p-nitrophenylphosphate as the substrate, MgCl₂ and protein phosphatase 1G are incubated, and generated p-nitrophenol is measured (absorbance at 410 nm is measured).

When glycogen synthase activity in the presence of an added candidate compound is higher than that in the absence of the added candidate compound and when the candidate compound does not exhibit any activity-modulating effect on the factors involved in the regulation of glycogen synthase activity, the candidate compound can be judged to be a compound directly activating glycogen synthase.

### (Therapeutic agent for diabetes comprising a compound directly activating glycogen synthase as an active ingredient)

According to the screening method of the present invention, a compound directly activating glycogen synthase can be selected. And the therapeutic agent for diabetes of the present invention comprises the compound as the active ingredient. For the dosage form of the therapeutic agent for diabetes of the present invention, various forms can be selected, and for example, oral formulation such as tablet, capsule, powder, granule, and liquid, sterilized liquid parenteral formulation such as solution and suspension, and the like can be listed.

Solid formulation can be produced in the form of tablet, capsule, granule, or powder either as it is or by using appropriate additives. The additives include, for example, sugars such as lactose and glucose, starches such as corn, wheat, and rice, fatty acids such as stearic acid, inorganic salts such as sodium metasilicate, magnesium aluminosilicate, and anhydrous calcium phosphate, synthetic polymers such as polyvinylpyrrolidone and polyalkylene glycol, fatty acid salts such as calcium stearate and magnesium stearate, alcohols such as stearyl alcohol and benzyl alcohol, synthetic cellulose derivatives such as methylcellulose, carboxymethylcellulose, ethylcellulose, and hydroxypropylmethylcellulose, and other commonly used additives such as water, gelatin, talc, vegetable oil, and gum Arabic.

These solid formulations such as tablet, capsule, granule, and powder may contain generally 0.1% to 100% by weight, preferably 5% to 100% by weight of the active ingredient. The liquid formulation can be produced in the form of suspension, syrup, injection, and the like using water, alcohols, or appropriate additives commonly used in the liquid formulation, for example, vegetable-derived oils such as soybean oil, peanut oil, and sesame oil. In particular, appropriate solvent in a case of parenteral administration includes, for example, distilled water for injection, an aqueous solution of lidocaine hydrochloride (for intramuscular injection), saline, an aqueous solution of glucose, ethanol, liquid for intravenous injection (for example, an aqueous solution of citric acid or sodium citrate), and an electrolyte solution (for example, for intravenous drip infusion or for intravenous injection), or a mixed solution thereof. The liquid formulation for oral administration such as suspension or syrup may contain 0.5% to 10% by weight of the active ingredient.

The practically preferred dosage of the compound directly activating glycogen synthase can be appropriately increased or decreased depending on the kind of compound used, the kind of ingredient mixed, frequency of application, specific site to be treated, and condition of patient. For example, the dose per adult is 0.1 to 1000 mg per day when administered orally and 0.01 to 500 mg per day when administered parenterally. It should be noted that although the number of doses differs depending on administration methods and conditions, dosing may be performed once or by dividing into 2 to 5 times.

### (Nucleic acid, vector, and protein)

The nucleic acid of the present invention comprises the nucleotide sequence according to SEQ ID NO:11 in Sequence Listing, and the vector of the present invention is inserted with the nucleic acid of the present invention. Further the protein of the present invention comprises the amino acid sequence according to SEQ ID NO:12. The nucleic acid of the present invention is not limited to the nucleotide sequence according to SEQ ID NO:11 and includes all nucleotide sequences that encode the amino acid sequence according to SEQ ID NO:12.

A nucleic acid comprising the nucleotide sequence according to SEQ ID NO:11 encodes a variant of human glycogen synthase 2 (protein comprising the amino acid sequence according to SEQ ID NO:12). Among 7 serine phosphorylation sites that glycogen synthase 2 has, six serine residues at sites 2, 3a, 3b, 3c, 4, and 5 (corresponding to 8th, 641st, 645th, 649th, 653rd, and 657th amino acid residues in SEQ ID NO:12, respectively) are substituted by alanine residues in this variant. This variant lacking the phosphorylation sites is not converted to an inactive form by being phosphorylated, stays in an active form at all times, and is always able to carry out glycogen synthesis without being affected by kinases present *in vivo.* Accordingly, gene therapy of diabetes can be performed using the nucleic acid of the present invention, and further the protein of the present invention can be used as a therapeutic agent for diabetes.

Another nucleic acid of the present invention comprises the nucleotide sequence according to SEQ ID NO:13 in Sequence Listing, and the vector of the present invention is inserted with the nucleic acid of the present invention. Further, the protein of the present invention comprises the amino acid sequence according to SEQ ID NO:14. The nucleic acid of the present invention is not limited to the nucleotide sequence according to SEQ ID NO:13 and includes all nucleotide sequences that encode the amino acid sequence according to SEQ ID NO:14.

The nucleic acid comprising the nucleotide sequence according to SEQ ID NO:13 encodes a variant of human glycogen synthase 2 (protein comprising the amino acid sequence according to SEQ ID NO:14). In this variant, all serine residues of the 7 serine phosphorylation sites that glycogen synthase 2 has (sites 2, 2a, 3a, 3b, 3c, 4, and 5) are substituted by alanine residues (corresponding to 8th, 11th, 641st, 645th, 649th, 653rd, and 657th amino acid residues in SEQ ID NO:14, respectively). This variant lacking the phosphorylation sites is not converted to an inactive form by being phosphorylated, stays in an active form at all times, and is always able to carry out glycogen synthesis without being affected by kinases present *in vivo.* Accordingly, gene therapy of diabetes can be performed using the nucleic acid of the present invention, and further the protein of the present invention can be used as a therapeutic agent for diabetes.

The nucleic acid of the present invention can be prepared by modifying cDNA of human glycogen synthase 2 using a site-directed mutagenesis method. More specifically, for example, PCR is performed first using human liver cDNA as a template, and the amplified product obtained is subcloned into an appropriate vector. Subsequently, the codon coding for the above-described serine residues is replaced by the codon coding for alanine with the use of a commercially available site-directed mutagenesis kit, thereby obtaining the nucleic acid of the present invention.

By inserting the nucleic acid of the present invention into a vector for therapy, it is possible to conduct gene therapy of diabetes. The vector for therapy is not particularly limited and may be either a virus vector or a non-virus vector (plasmid vector). For the virus vector, for example, recombinant virus vectors derived from adenovirus, adeno-associated virus, herpes virus, HIV virus, Sendai virus, and the like can be used. Into the above vector, sequences involved in phenotypic expression such as appropriate promoter, replication origin, selection marker, RNA splice sites, and polyadenylation signal are introduced.

The nucleic acid of the present invention incorporated into the above vector can be used as an agent for gene therapy according to a conventional technique. That is, a recombinant virus vector may be contacted with a therapeutic target cell (for example, hepatocyte) or a plasmid vector may be introduced into the target cell by the calcium phosphate method, liposome method, electroporation method, or the like.

The protein of the present invention can be obtained by incorporating the nucleic acid of the present invention into a vector, introducing this into a host such as *Escherichia coli,* then allowing the nucleic acid to be expressed by culturing the host cells in a medium. After culturing the host cells, the cells are lysed as necessary, and then the protein of the present invention can be purified by conventional techniques such as salting out, dialysis, ultrafiltration, gel filtration, various kinds of chromatography (ionexchange chromatography, affinity chromatography, etc.), and electrophoresis.

The protein of the present invention can be used as a therapeutic agent for diabetes. That is, the therapeutic agent for diabetes of the present invention comprises the protein of the present invention as an active ingredient. Although the dosage form of the therapeutic agent for diabetes is not particularly limited, mainly parenteral dosage form such as injection is common. Formulation can be carried out according to a conventional method, and the therapeutic agent for diabetes may contain additives such as stabilizer, solubilizing agent, antiseptic agent, and antioxidant as needed.

A preferred dose of the protein of the present invention can be appropriately increased or decreased depending on the condition of patient and the like. For example, in the case of parenteral administration, the daily dose per adult is 1 µg to 100 mg per day as the protein of the present invention. In addition, the number of doses varies depending on dosing method and condition, and dosing can be performed once or by dividing into 2 to 5 times.

### Examples

Hereinafter, the present invention is explained in more detail with reference to examples. However, the present invention is not limited to these examples.

### (Production example 1: Construction of pcDNA3.1 (-)/AS-SA GYS2 plasmid)

### (1) Cloning of glycogen synthase 2 (GYS2) gene

PCR was performed using PfuTurbo DNA polymerase (STRATAGENE) with human liver cDNA (liver cDNA (S-1202), Multiple tissue cDNA panel, CLONTEC) as a template and GYS-2-FW (SEQ ID NO:1) and GYS-2-RV (SEQ ID NO:2) as primers (94°C for 1 min, 60°C for 1 min, 72°C for 3 min, 25 cycles). The amplified fragment obtained was treated with HindIII-EcoRI and then subcloned into pFLAGCTC (SIGMA). The obtained plasmid was designated as pFLAGCTC/GYS2. The sequences of the primers used are as follows.
GYS-2-FW: catatgaagcttcgaggccgatccctctctgta
GYS-2-RV: acccgggaattcgttcttatattcaccatgcagctt

PCR was performed using pFLAGCTC/GYS2 as a template and GYS2-V-FW (SEQ ID NO:3) and GYS2-V-RV (SEQ ID NO:4) as primers. The amplified fragment obtained was treated with EcoRI-HindIII and then subcloned into pcDNA3.1 (-). The obtained plasmid was designated as pcDNA3.1(-)/GYS2. The sequences of the primers used are as follows.
GYS-V FW: ttttttgaattccgccaccatgcttcgaggccgatccctctctgtaac
GYS-V RV: ttttttaagctttcagttcttatattcaccatgcagc

### (2) Substitution of Ala for Ser at site 2

PCR was performed using pcDNA3.1(-)/GYS2 as a template and GYS2-V-FW-Site 2 (SEQ ID NO:5) and GYS2-V-RV-Site 2 (SEQ ID NO:6) as primers. The amplified fragment obtained was treated with EcoRI-HindIII and then subcloned into pcDNA3.1 (-). The obtained plasmid was designated as pcDNA3.1(-)/AS-5S GYS2. The sequences of the primers used are as follows.
GYS2-V-FW-Site2: ttttttgaattccgccaccatgcttcgaggccgatccctcgctgtaacatcc (The underlined portion corresponds to site 2)
GYS2-V RV Site2: ttttttaagctttcagttcttatattcaccatgcagc

### (3) Substitution of Ala for Ser at sites 3a and 3b

Mutations were introduced using QuickChange Site-Directed Mutagenesis Kit (STRATGENE) with pcDNA3.1(-)/AS-5S GYS2 as a template and GYS2-3a, 3b-sense (SEQ ID NO:7) and GYS2-3a, 3b-antisense (SEQ ID NO:8) as primers. The obtained plasmid was designated as pcDNA3.1(-)/AS-AASSS GYS2. The sequences of the primers used are as follows.
GYS2-3a,3b-sense: agaaggatttaaatatcccaggccttccgctgtaccacctgctccttcaggg (The underlined portions correspond to sites 3a and 3b in order)
GYS2-3a,3b-antisense: ccctgaaggagcaggtggtacagcggaaggcctgggatatttaaatccttct (The underlined portions correspond to sites 3b and 3a in order)

### (4) Substitution of Ala for Ser at sites 3c, 4, and 5

Mutations were introduced using QuickChange Site-Directed Mutagenesis Kit with pcDNA3.1(-)/AS-AASSS GYS2 as a template and GYS2-3c, 4, 5-sense (SEQ ID NO:9) and GYS2-3c, 4, 5-antisense (SEQ ID NO:10) as primers. The obtained plasmid was designated as pcDNA3.1(-)/AS-5A GYS2. The sequences of the primers used are as follows.
GYS2-3c,4,5-sense: cctgctccttcaggggctcaggcctccgctcctcagagcgctgatgtggaagatgaag (The underlined portions correspond to sites 3c, 4, and 5 in order)
GYS2-3c,4,5-antisense: cttcatcttccacatcagcgctctgaggagcggaggcctgagcccctgaaggagcagg (The underlined portions correspond to sites 5, 4, and 3c in order)

The plasmid pcDNA3.1(-)/AS-5A GYS2 obtained by the above procedures has an open reading frame (ORF) of a variant of human GYS2 in which the codons coding for sites 2, 3a, 3b, 3c, 4, and 5 among serine phosphorylation sites in ORF of human GYS2 were substituted by GCTs (Ala codon).

### (Production example 2: Construction of pcDNA3.1(-)/AA-5A GYS2 plasmid)

### (1) Substitution of Ala for Ser at sites 2 and 2a

PCR was performed using the pcDNA3.1(-)/GYS2 obtained in Production example 1 as a template and GYS2-V-FW-S2, 2a (SEQ ID NO:15) and GYS2-V-RV-S2, 2a (SEQ ID NO:16) as primers. The amplified fragment obtained was treated with EcoRI-HindIII and then subcloned into pcDNA3.1(-). The obtained plasmid was designated as pcDNA3.1(-)/AA-5S GYS2. The sequences of the primers used are as follows.
GYS2-V FW S2,2a: ttttttgaattccgccaccatgcttcgaggccgatccctcgctgtaacagctctgggtggg (The underlined portions correspond to sites 2 and 2a in order)
GYS2-V RV S2,2a: ttttttaagctttcagttcttatattcaccatgcagc

### (2) Substitution of Ala for Ser at sites 3a and 3b and substitution of Ala for Ser at sites 3c, 4, and 5

Ser residues at sites 3a, 3b, 3c, 4, and 5 were substituted by Ala by a method similar to that described in (3) and (4) of Production example 1 using pcDNA3.1(-)/AA-5S GYS2 as a template. The obtained plasmid was designated as pcDNA3.1(-)/AA-5A GYS2. This plasmid has an open reading frame (ORF) of a variant of human GYS2 in which the codons coding for all of the serine phosphorylation sites (sites 2, 2a, 3a, 3b, 3c, 4, and 5) in open reading frame (ORF) of human GYS2 were substituted by GCTs (Ala codon).

### (Production example 3: Construction of recombinant adenovirus)

First, an adenovirus vector, pCosAdv-CMV, was constructed by the following method. A genome sequence derived from an adenovirus lacking E1 and E3 regions was prepared according to the method of Morsy et al. (J. Clin. Invest., 92, 1580-1586 (1993)). A restriction site (SwaI) for insertion of a target gene was introduced between the CMV promoter and BGH-polyA addition signal sequence. PacI and PmeI sites were inserted into a cosmid vector, Charomid 9-20 (WAKO), and the genome sequence derived from the adenovirus was inserted at the PacI site, thus constructing the pCosAdv-CMV vector.

The pCosAdv-CMV was digested with SwaI, and the terminus thereof was dephosphorylated by CIAP (alkaline phosphatase derived from calf intestine). The pcDNA3.1 (-)/AS-5A GYS2 and pcDNA3.1(-)/AA-5A GYS2 constructed in Production examples 1 and 2 were treated with EcoRV-HindIII, and the obtained DNA fragments were blunted by Klenow fragment and subcloned into the adenovirus vector. Transformation of *E. coli* was performed by using In vitro Packaging Kit LAMBDA INN (NIPPON GENE), and recombinant adenovirus plasmids in which the target genes were inserted in the right direction were purified. The purified plasmid DNAs were completely digested with PacI to cut out virus DNA, followed by transfection into 293 cells. After the transfection, the 293 cells were recovered, frozen and thawed, and centrifuged to obtain the supernatant as a virus solution. Further, amplification of the virus was repeated by infecting 293 cells with this virus solution, and a large amount of the virus solution (for each of Ad-AS-5A and Ad-AA-5A) was obtained. As a control, an adenovirus vector inserted with lacZ (Ad-lacZ) was constructed, and a large amount of the virus solution was obtained in a similar way. Further, an adenovirus vector inserted with pcDNA3.1(-)/GYS2 was constructed for comparison, and a large amount of the virus solution was obtained in a similar way.

The obtained virus solution was purified by CsCl density gradient centrifugation and dialyzed against a dialysis buffer (10 mM Tris-HCl (pH 7.5), 1 mM MgCl₂, 10% glycerol). The recovered virus solution was stored at -80°C until use.

### (Test example 1: Assay of amount of glucose production)

Hepatocytes were isolated from rats (7- to 9-week-old, male) by the portal perfusion method and suspended in a high glucose DMEM (GIBCO) containing 10% FBS (fetal bovine serum), and the cells were seeded in a 100 mm collagen-coated dish (IWAKI) at a density of 3.5X10⁶ cell/10 mL. After 3 hours, the medium was replaced with a serum-free low glucose DMEM, and the recombinant adenovirus was allowed to infect the cells for 2 hours. This medium was replaced with a low glucose DMEM containing 10% FBS, followed by culturing overnight.

The medium was replaced with a glucose-free DMEM containing 10 mM dihydroxyacetone and incubated for 3 to 6 hours, and then the amount of glucose released into the medium was measured with Determiner GL-E (KYOWA). This measured value corresponds to the amount of glucose production mainly from dihydroxyacetone that is a substrate precursor for gluconeogenesis, namely, the amount of glucose production by gluconeogenesis.

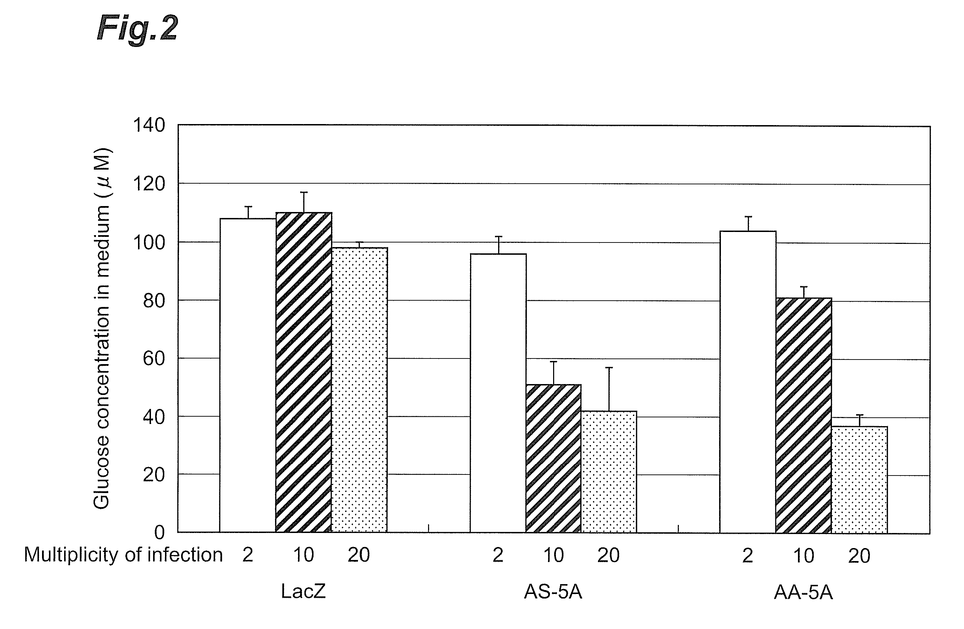

Figure 2 is a graph showing the amounts of glucose production by the control (LacZ) and the variants of GYS2 (AS-5A and AA-5A) at various multiplicities of infection. From Figure 2, it is understood that the amount of glucose production is reduced in both variants of GYS2 compared to the control. When the multiplicities of infection were 2, 10, and 20, the rates of reduction in glucose production based on the control were 11 %, 54%, and 58% respectively in the variant of GYS2 (AS-5A) and 4%, 26%, and 62% respectively in the variant of GYS2 (AA-5A).

On the other hand, when the wild-type GYS2 was over-expressed, the amount of glucose production showed little change even compared to the control. That is, when the multiplicities of infection were 2, 10, and 20, the rates of reduction in glucose production were 15%, 13%, and 9% respectively.

The result that the amount of glucose production showed little change even when the wild-type GYS2 was over-expressed suggests that glycogen synthase is inactivated by being phosphorylated by glycogen synthase kinase 3 and the like. On the other hand, it is thought that the variants of GYS2 (AS-5A and AA-5A) are not inactivated even when glycogen synthase kinase 3 and the like are present because of the absence of phosphorylation sites and therefore the amount of glucose production is reduced.

### (Test example 2)

An oral glucose tolerance test was carried out under the condition that the variant of GYS2 (AS-5A) was over-expressed in mouse liver using the adenovirus vector, thereby enhancing the activity of liver GYS2, and whether or not glucose tolerance was improved was studied.

Each adenovirus was diluted with PBS as a vehicle so as to be adjusted to 5x10⁹ pfu/mouse. An immunosuppressive agent was prepared by the following procedure. Prednisone was made hydrophilic by adding ethanol (3.75% of the final volume), and cyclosporin A (Sandimmune injection) was added therein and mixed well. Then, sterile saline was added until reaching the final volume and further mixed.

### (1) Start of administration of immunosuppressive agent

The intraperitoneal administration of the immunosuppressive agent once a day was started from 2 days before administration of adenovirus. Two days and one day before the administration of adenovirus, 2.5 mg/kg of prednisone plus 8 mg/kg of cyclosporin A were administered, and on the day of the administration of adenovirus, 2.5 mg/kg of prednisone plus 16 mg/kg of cyclosporin A were administered. In addition, one day and 2 days after the administration of adenovirus, 2.5 mg/kg of prednisone plus 4 mg/kg of cyclosporin A were administered.

### (2) Administration of adenovirus

On the previous day of the administration of adenovirus, blood sugar level and body weight of a mouse under conditions of free access to food and water were assayed. First, using a capillary treated with heparin, a small amount of blood was collected from the orbital vein of the mouse under conscious conditions, and blood sugar level was assayed with Antsense II (BAYER MEDICAL) calibrated with standards.

On the day of the administration of adenovirus, the mouse was placed in a holder, and vehicle and Ad-LacZ or Ad-AS-5A (constructed in Production example 3) was administered intravenously from the tail vein by using a syringe with a 29G needle. The dose of adenovirus was 5X10⁹ pfu/mouse in each case.

### (3) Two days after administration: Assay of blood sugar level

The blood sugar levels in all mice were assayed on the morning of the second day after the administration under conditions of free access to food and water. Each mouse was placed in a holder, one drop of blood was collected from the tail, and the blood sugar level was assayed with Antsense II calibrated with standards.

### (4) Three days after administration: Oral glucose tolerance test

The body weights of fasting mice were assayed on the morning of the third day after the administration, and the amount of glucose to be given in the oral glucose tolerance test (1 g/10 mL/kg) performed in the afternoon was determined. In the afternoon, blood collection was carried out from the mouse tail, and blood sugar level immediately before the test was assayed. Next, a solution of 1 g/10 mL glucose (an aqueous solution of 0.5% methylcellulose as the vehicle) was administered orally at 10 mL/kg, and blood sugar levels were assayed after 30, 60, 90, and 120 min. The assay method of blood sugar level is as described in the above (3).

### (5) Assay of plasma glucose

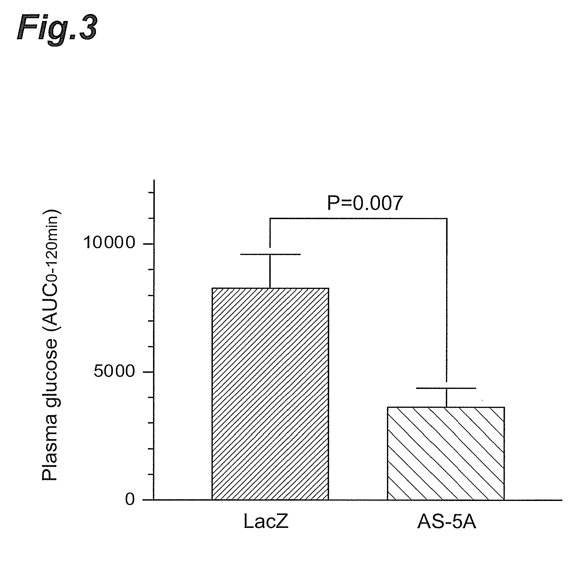

Plasma glucose was assayed using a glucose assay kit, "Determiner GL-E" (KYOWA MEDICS). Five microliters of plasma was diluted 20-fold with saline, and 20 µL each of standards and the sample was dispensed into a 96-well plate. An equal volume of Reagent 1 and Reagent 2 were mixed, and 150 µL of this mixture was dispensed into each well and incubated for 30 min at 37°C, followed by measurement of absorbance at 585 nm. The absorbance was measured using SPECTRAMAX 340PC (MOLECULAR DEVICE Co.) and analyzed with the use of software, SOFTmaxPro3.1.1, attached to the instrument. The obtained result was shown by AUC (the Area Under the blood concentration-time Curve; 0 to 120 min) (Figure 3). As is evident from Figure 3, glucose tolerance was improved in the mouse with high expression of AS-5S.

### Industrial Applicability

According to the screening method of the present invention, it is possible to obtain compounds directly activating glycogen synthase. These compounds can be used for treatment of diabetes.

## Claims

1. A therapeutic agent for diabetes comprising a compound directly activating glycogen synthase as an active ingredient.

2. A method for screening compounds directly activating glycogen synthase, the method comprising the steps of:
(a) allowing glycogen synthase and a candidate compound to contact each other to assay glycogen synthase activity under the condition that glucose-6-phosphatase, hexokinase, glycogen phosphorylase, glycogen synthase kinase 3, and protein phosphatase 1G are not present; and
(b) selecting a candidate compound having an activity assayed in the step (a) higher than that assayed in the same manner as in the step (a) except that the candidate compound is not used.

3. A method for screening compounds directly activating glycogen synthase, the method comprising the steps of:
(a) allowing glycogen synthase and a candidate compound to contact each other to assay glycogen synthase activity;
(b) measuring the binding constant between glycogen synthase and the candidate compound; and
(c) selecting a candidate compound showing an activity in the step (a) higher than that assayed in the same manner as in the step (a) except that the candidate compound is not used and having a binding constant equal to or larger than a predetermined value.

4. A method for screening compounds directly activating glycogen synthase, the method comprising the steps of:
(a) allowing glycogen synthase and a candidate compound to contact to each other to assay glycogen synthase activity;
(b) assaying an activity-modulating effect of the candidate compound on at least one enzyme selected from the group consisting of glucose-6-phosphatase, hexokinase, glycogen phosphorylase, glycogen synthase kinase 3, and protein phosphatase 1G; and
(c) selecting a candidate compound showing an activity in the step (a) higher than that assayed in the same manner as in the step (a) except that the candidate compound is not used and not showing any activity-modulating effect in the step (b).

5. The method for screening according to any one of Claims 2 to 4, wherein the glycogen synthase is glycogen synthase 2.

6. An isolated nucleic acid comprising the nucleotide sequence according to SEQ ID NO:11.

7. A vector inserted with a nucleic acid according to Claim 6.

8. The vector according to Claim 7, wherein the vector is for gene therapy.

9. An isolated protein comprising the amino acid sequence according to SEQ ID NO:12.

10. A therapeutic agent for diabetes comprising a protein according to Claim 9 as an active ingredient.

11. An isolated nucleic acid comprising the nucleotide sequence according to SEQ ID NO:13.

12. A vector inserted with a nucleic acid according to Claim 11.

13. The vector according to Claim 12, wherein the vector is for gene therapy

14. An isolated protein comprising the amino acid sequence according to SEQ ID NO:14.

15. A therapeutic agent for diabetes comprising a protein according to Claim 14 as an active ingredient.


SEQUENCE LISTING

<110>  Banyu Pharmaceutical Co., Ltd.

<120>  COMPOUND DIRECTLY ACTIVATING GLYCOGEN SYNTHASE

<130>  FP05-0241-00

<150>  JP 2004-188,711
<151>  2005-06-25

<160>  16

<170>  PatentIn version 3.1

<210>  1
<211>  33
<212>  DNA
<213>  Artificial

<220>
<223>  GYS-2-FW

<400>  1
catatgaagc ttcgaggccg atccctctct gta                                    33


<210>  2
<211>  36
<212>  DNA
<213>  Artificial

<220>
<223>  GYS-2-RV

<400>  2
acccgggaat tcgttcttat attcaccatg cagctt                                 36


<210>  3
<211>  48
<212>  DNA
<213>  Artificial

<220>
<223>  GYS2-V-FW

<400>  3
ttttttgaat tccgccacca tgcttcgagg ccgatccctc tctgtaac                     48


<210>  4
<211>  37
<212>  DNA
<213>  Artificial

<220>
<223>  GYS2-V-RV

<400>  4
ttttttaagc tttcagttct tatattcacc atgcagc                                 37


<210>  5
<211>  52
<212>  DNA
<213>  Artificial

<220>

<223>  GYS2-V-FW-Site2

<400>  5
ttttttgaat tccgccacca tgcttcgagg ccgatccctc gctgtaacat cc          52


<210>  6
<211>  37
<212>  DNA
<213>  Artificial

<220>
<223>  GYS2-V-RV

<400>  6
ttttttaagc tttcagttct tatattcacc atgcagc                          37


<210>  7
<211>  52
<212>  DNA
<213>  Artificial

<220>
<223>  GYS2-3a,3b-sense

<400>  7
agaaggattt aaatatccca ggccttccgc tgtaccacct gctccttcag gg          52


<210>  8
<211>  52
<212>  DNA
<213>  Artificial

<220>
<223>  GYS2-3a,3b-antisense

<400>  8
ccctgaagga gcaggtggta cagcggaagg cctgggatat ttaaatcctt ct          52


<210>  9
<211>  58
<212>  DNA
<213>  Artificial

<220>
<223>  GYS2-3c,4,5-sense

<400>  9
cctgctcctt caggggctca ggcctccgct cctcagagcg ctgatgtgga agatgaag    58


<210>  10
<211>  58
<212>  DNA
<213>  Artificial

<220>
<223>  GYS2-3c,4,5-antisense

<400>  10
cttcatcttc cacatcagcg ctctgaggag cggaggcctg agcccctgaa ggagcagg    58


<210>  11
<211>  2112
<212>  DNA

<213>  Artificial

<220>
<223>  human GYS2 AS-5A

<220>
<221>  CDS
<222>  (1)..(2112)
<223>

<400>  11
atg ctt cga ggc cga tcc ctc gct gta aca tcc ctg ggt ggg ctt ccc      48
Met Leu Arg Gly Arg Ser Leu Ala Val Thr Ser Leu Gly Gly Leu Pro
1               5                   10                  15

cag tgg gaa gtc gaa gaa ctt cct gtg gag gag tta ctg ctc ttt gaa      96
Gln Trp Glu Val Glu Glu Leu Pro Val Glu Glu Leu Leu Leu Phe Glu
            20                  25                  30

gtt gct tgg gaa gtg acc aat aaa gtt gga ggc atc tat act gtg att      144
Val Ala Trp Glu Val Thr Asn Lys Val Gly Gly Ile Tyr Thr Val Ile
        35                  40                  45

cag aca aag gcc aaa aca aca gca gat gaa tgg gga gag aac tat ttt      192
Gln Thr Lys Ala Lys Thr Thr Ala Asp Glu Trp Gly Glu Asn Tyr Phe
    50                  55                  60

ctg ata ggt cca tat ttt gag cat aat atg aag act cag gtg gaa cag      240
Leu Ile Gly Pro Tyr Phe Glu His Asn Met Lys Thr Gln Val Glu Gln
65                  70                  75                  80

tgt gaa cct gta aat gat gct gtc aga aga gca gtg gac gca atg aat      288
Cys Glu Pro Val Asn Asp Ala Val Arg Arg Ala Val Asp Ala Met Asn
                85                  90                  95

aag cat ggc tgc cag gtg cat ttt gga aga tgg ctg ata gaa gga agt      336
Lys His Gly Cys Gln Val His Phe Gly Arg Trp Leu Ile Glu Gly Ser
            100                 105                 110

cct tat gtg gta ctt ttt gac ata ggc tat tca gct tgg aat ctg gac      384
Pro Tyr Val Val Leu Phe Asp Ile Gly Tyr Ser Ala Trp Asn Leu Asp
        115                 120                 125

agg tgg aag ggt gac ctc tgg gaa gca tgc agt gtc ggc att cct tat      432
Arg Trp Lys Gly Asp Leu Trp Glu Ala Cys Ser Val Gly Ile Pro Tyr
    130                 135                 140

cat gac cga gaa gcc aat gat atg ctg ata ttt gga tct tta act gcc      480
His Asp Arg Glu Ala Asn Asp Met Leu Ile Phe Gly Ser Leu Thr Ala
145                 150                 155                 160

tgg ttc tta aaa gag gtg aca gat cat gca gat ggt aaa tat gtc gtt      528
Trp Phe Leu Lys Glu Val Thr Asp His Ala Asp Gly Lys Tyr Val Val
                165                 170                 175

gcc caa ttc cat gaa tgg cag gct gga att gga ctg atc ctt tct cga      576
Ala Gln Phe His Glu Trp Gln Ala Gly Ile Gly Leu Ile Leu Ser Arg
            180                 185                 190

gcc agg aaa ctt cct att gcc aca ata ttt aca acc cac gct aca cta      624
Ala Arg Lys Leu Pro Ile Ala Thr Ile Phe Thr Thr His Ala Thr Leu
        195                 200                 205

ctt ggg agg tat ctc tgt gca gca aat att gat ttc tac aac cat ctt      672
Leu Gly Arg Tyr Leu Cys Ala Ala Asn Ile Asp Phe Tyr Asn His Leu
    210                 215                 220

gat aag ttt aac att gac aaa gag gct ggg gaa agg cag att tac cac      720
Asp Lys Phe Asn Ile Asp Lys Glu Ala Gly Glu Arg Gln Ile Tyr His

225                 230                 235                 240

cgg tac tgc atg gag cga gct tcc gtt cat tgc gct cac gtg ttc acc       768
Arg Tyr Cys Met Glu Arg Ala Ser Val His Cys Ala His Val Phe Thr
                245                 250                 255

acg gtt tct gaa ata aca gca ata gaa gct gaa cat atg ctg aag aga       816
Thr Val Ser Glu Ile Thr Ala Ile Glu Ala Glu His Met Leu Lys Arg
            260                 265                 270

aag cct gat gta gtt act cca aac ggc ttg aat gtt aag aaa ttt tca       864
Lys Pro Asp Val Val Thr Pro Asn Gly Leu Asn Val Lys Lys Phe Ser
        275                 280                 285

gca gtg cat gag ttt caa aat cta cat gcc atg tac aag gcc aga atc       912
Ala Val His Glu Phe Gln Asn Leu His Ala Met Tyr Lys Ala Arg Ile
    290                 295                 300

caa gat ttt gtt cga ggt cat ttc tat ggt cat ctc gac ttt gat ctt       960
Gln Asp Phe Val Arg Gly His Phe Tyr Gly His Leu Asp Phe Asp Leu
305                 310                 315                 320

gaa aag act ttg ttc ctt ttc att gct ggg agg tat gag ttt tca aac      1008
Glu Lys Thr Leu Phe Leu Phe Ile Ala Gly Arg Tyr Glu Phe Ser Asn
                325                 330                 335

aaa gga gct gac atc ttc cta gaa tcc tta tcc agg cta aat ttc ctg      1056
Lys Gly Ala Asp Ile Phe Leu Glu Ser Leu Ser Arg Leu Asn Phe Leu
            340                 345                 350

ctg agg atg cat aaa agt gac atc aca gtg gtg gtg ttt ttc att atg      1104
Leu Arg Met His Lys Ser Asp Ile Thr Val Val Val Phe Phe Ile Met
        355                 360                 365

cct gcc aag aca aat aat ttc aac gtg gaa acc ctg aaa gga caa gca      1152
Pro Ala Lys Thr Asn Asn Phe Asn Val Glu Thr Leu Lys Gly Gln Ala
    370                 375                 380

gtg cga aaa cag ctg tgg gat gtt gca cat tct gtg aag gaa aag ttt      1200
Val Arg Lys Gln Leu Trp Asp Val Ala His Ser Val Lys Glu Lys Phe
385                 390                 395                 400

gga aaa aaa ctc tat gat gca tta tta aga gga gaa att cct gac ctg      1248
Gly Lys Lys Leu Tyr Asp Ala Leu Leu Arg Gly Glu Ile Pro Asp Leu
                405                 410                 415

aac gat att tta gat cga gat gat cta aca att atg aaa aga gcc atc      1296
Asn Asp Ile Leu Asp Arg Asp Asp Leu Thr Ile Met Lys Arg Ala Ile
            420                 425                 430

ttt tca act cag cga cag tca ttg ccc cca gtg acc acg cac aac atg      1344
Phe Ser Thr Gln Arg Gln Ser Leu Pro Pro Val Thr Thr His Asn Met
        435                 440                 445

att gat gac tcc acc gac ccc atc ctc agc acc att aga cgg att gga      1392
Ile Asp Asp Ser Thr Asp Pro Ile Leu Ser Thr Ile Arg Arg Ile Gly
    450                 455                 460

ctt ttc aac aac cgc aca gat aga gtc aag gtg att ttg cac cca gag      1440
Leu Phe Asn Asn Arg Thr Asp Arg Val Lys Val Ile Leu His Pro Glu
465                 470                 475                 480

ttt cta tcc tcc acc agt ccc tta cta ccc atg gac tat gaa gag ttt      1488
Phe Leu Ser Ser Thr Ser Pro Leu Leu Pro Met Asp Tyr Glu Glu Phe
                485                 490                 495

gtt aga ggt tgt cat ctt gga gta ttt cca tca tac tat gaa ccc tgg      1536
Val Arg Gly Cys His Leu Gly Val Phe Pro Ser Tyr Tyr Glu Pro Trp
            500                 505                 510

ggt tat act cca gct gaa tgc act gtg atg ggt atc ccc agt gtg acc 1584
Gly Tyr Thr Pro Ala Glu Cys Thr Val Met Gly Ile Pro Ser Val Thr
        515                 520                 525

acg aat ctc tcc ggg ttt ggc tgt ttc atg cag gag cac gtg gct gat 1632
Thr Asn Leu Ser Gly Phe Gly Cys Phe Met Gln Glu His Val Ala Asp
    530                 535                 540

cct act gct tac ggt att tac atc gtt gac agg cgg ttc cgt tct cca 1680
Pro Thr Ala Tyr Gly Ile Tyr Ile Val Asp Arg Arg Phe Arg Ser Pro
545                 550                 555                 560

gat gat tct tgc aat cag ctg act aag ttt ctc tat gga ttt tgc aaa 1728
Asp Asp Ser Cys Asn Gln Leu Thr Lys Phe Leu Tyr Gly Phe Cys Lys
                565                 570                 575

cag tca cgc cgc caa agg att atc cag agg aac aga act gag agg ctc 1776
Gln Ser Arg Arg Gln Arg Ile Ile Gln Arg Asn Arg Thr Glu Arg Leu
            580                 585                 590

tca gat ctt ctg gat tgg aga tac tta ggc aga tat tac cag cat gcc 1824
Ser Asp Leu Leu Asp Trp Arg Tyr Leu Gly Arg Tyr Tyr Gln His Ala
        595                 600                 605

aga cac ctg aca tta agc aga gct ttt cca gat aaa ttc cat gtg gaa 1872
Arg His Leu Thr Leu Ser Arg Ala Phe Pro Asp Lys Phe His Val Glu
    610                 615                 620

cta aca tca cca cca acg aca gaa gga ttt aaa tat ccc agg cct tcc 1920
Leu Thr Ser Pro Pro Thr Thr Glu Gly Phe Lys Tyr Pro Arg Pro Ser
625                 630                 635                 640

gct gta cca cct gct cct tca ggg gct cag gcc tcc gct cct cag agc 1968
Ala Val Pro Pro Ala Pro Ser Gly Ala Gln Ala Ser Ala Pro Gln Ser
                645                 650                 655

gct gat gtg gaa gat gaa gtg gag gat gag aga tac gat gag gaa gag 2016
Ala Asp Val Glu Asp Glu Val Glu Asp Glu Arg Tyr Asp Glu Glu Glu
            660                 665                 670

gag gct gaa agg gat cgg tta aat atc aag tca cca ttt tca ctg agc 2064
Glu Ala Glu Arg Asp Arg Leu Asn Ile Lys Ser Pro Phe Ser Leu Ser
        675                 680                 685

cac gtt cct cat ggg aag aaa aag ctg cat ggt gaa tat aag aac tga 2112
His Val Pro His Gly Lys Lys Lys Leu His Gly Glu Tyr Lys Asn
    690                 695                 700

<210> 12
<211> 703
<212> PRT
<213> Artificial

<220>
<223> human GYS2 AS-5A

<400> 12

Met Leu Arg Gly Arg Ser Leu Ala Val Thr Ser Leu Gly Gly Leu Pro
1               5                   10                  15

Gln Trp Glu Val Glu Glu Leu Pro Val Glu Glu Leu Leu Leu Phe Glu
            20                  25                  30

Val Ala Trp Glu Val Thr Asn Lys Val Gly Gly Ile Tyr Thr Val Ile

35                  40                  45

Gln Thr Lys Ala Lys Thr Thr Ala Asp Glu Trp Gly Glu Asn Tyr Phe
    50                  55                  60

Leu Ile Gly Pro Tyr Phe Glu His Asn Met Lys Thr Gln Val Glu Gln
65                  70                  75                  80

Cys Glu Pro Val Asn Asp Ala Val Arg Arg Ala Val Asp Ala Met Asn
                85                  90                  95

Lys His Gly Cys Gln Val His Phe Gly Arg Trp Leu Ile Glu Gly Ser
            100                 105                 110

Pro Tyr Val Val Leu Phe Asp Ile Gly Tyr Ser Ala Trp Asn Leu Asp
        115                 120                 125

Arg Trp Lys Gly Asp Leu Trp Glu Ala Cys Ser Val Gly Ile Pro Tyr
    130                 135                 140

His Asp Arg Glu Ala Asn Asp Met Leu Ile Phe Gly Ser Leu Thr Ala
145                 150                 155                 160

Trp Phe Leu Lys Glu Val Thr Asp His Ala Asp Gly Lys Tyr Val Val
                165                 170                 175

Ala Gln Phe His Glu Trp Gln Ala Gly Ile Gly Leu Ile Leu Ser Arg
            180                 185                 190

Ala Arg Lys Leu Pro Ile Ala Thr Ile Phe Thr Thr His Ala Thr Leu
        195                 200                 205

Leu Gly Arg Tyr Leu Cys Ala Ala Asn Ile Asp Phe Tyr Asn His Leu
    210                 215                 220

Asp Lys Phe Asn Ile Asp Lys Glu Ala Gly Glu Arg Gln Ile Tyr His
225                 230                 235                 240

Arg Tyr Cys Met Glu Arg Ala Ser Val His Cys Ala His Val Phe Thr
                245                 250                 255

Thr Val Ser Glu Ile Thr Ala Ile Glu Ala Glu His Met Leu Lys Arg
            260                 265                 270

Lys Pro Asp Val Val Thr Pro Asn Gly Leu Asn Val Lys Lys Phe Ser
        275                 280                 285

Ala Val His Glu Phe Gln Asn Leu His Ala Met Tyr Lys Ala Arg Ile
    290                 295                 300

Gln Asp Phe Val Arg Gly His Phe Tyr Gly His Leu Asp Phe Asp Leu
305                 310                 315                 320

Glu Lys Thr Leu Phe Leu Phe Ile Ala Gly Arg Tyr Glu Phe Ser Asn
                325                 330                 335

Lys Gly Ala Asp Ile Phe Leu Glu Ser Leu Ser Arg Leu Asn Phe Leu
            340                 345                 350

Leu Arg Met His Lys Ser Asp Ile Thr Val Val Val Phe Phe Ile Met
        355                 360                 365

Pro Ala Lys Thr Asn Asn Phe Asn Val Glu Thr Leu Lys Gly Gln Ala
    370                 375                 380

Val Arg Lys Gln Leu Trp Asp Val Ala His Ser Val Lys Glu Lys Phe
385                 390                 395                 400

Gly Lys Lys Leu Tyr Asp Ala Leu Leu Arg Gly Glu Ile Pro Asp Leu
                405                 410                 415

Asn Asp Ile Leu Asp Arg Asp Asp Leu Thr Ile Met Lys Arg Ala Ile
            420                 425                 430

Phe Ser Thr Gln Arg Gln Ser Leu Pro Pro Val Thr Thr His Asn Met
        435                 440                 445

Ile Asp Asp Ser Thr Asp Pro Ile Leu Ser Thr Ile Arg Arg Ile Gly
    450                 455                 460

Leu Phe Asn Asn Arg Thr Asp Arg Val Lys Val Ile Leu His Pro Glu
465                 470                 475                 480

Phe Leu Ser Ser Thr Ser Pro Leu Leu Pro Met Asp Tyr Glu Glu Phe
                485                 490                 495

Val Arg Gly Cys His Leu Gly Val Phe Pro Ser Tyr Tyr Glu Pro Trp
            500                 505                 510

Gly Tyr Thr Pro Ala Glu Cys Thr Val Met Gly Ile Pro Ser Val Thr
        515                 520                 525

Thr Asn Leu Ser Gly Phe Gly Cys Phe Met Gln Glu His Val Ala Asp
    530                 535                 540

Pro Thr Ala Tyr Gly Ile Tyr Ile Val Asp Arg Arg Phe Arg Ser Pro
545                 550                 555                 560

Asp Asp Ser Cys Asn Gln Leu Thr Lys Phe Leu Tyr Gly Phe Cys Lys
                565                 570                 575

Gln Ser Arg Arg Gln Arg Ile Ile Gln Arg Asn Arg Thr Glu Arg Leu
            580                 585                 590

Ser Asp Leu Leu Asp Trp Arg Tyr Leu Gly Arg Tyr Tyr Gln His Ala
        595                 600                 605

Arg His Leu Thr Leu Ser Arg Ala Phe Pro Asp Lys Phe His Val Glu
    610                 615                 620

Leu Thr Ser Pro Pro Thr Thr Glu Gly Phe Lys Tyr Pro Arg Pro Ser
625                 630                 635                 640

Ala Val Pro Pro Ala Pro Ser Gly Ala Gln Ala Ser Ala Pro Gln Ser
                645                 650                 655

Ala Asp Val Glu Asp Glu Val Glu Asp Glu Arg Tyr Asp Glu Glu Glu
            660                 665                 670

Glu Ala Glu Arg Asp Arg Leu Asn Ile Lys Ser Pro Phe Ser Leu Ser
        675                 680                 685

His Val Pro His Gly Lys Lys Lys Leu His Gly Glu Tyr Lys Asn
    690                 695                 700

<210>  13
<211>  2112
<212>  DNA
<213>  Artificial

<220>
<223>  human GYS2 AA-5A

<220>
<221>  CDS
<222>  (1)..(2112)
<223>

<400>  13
atg ctt cga ggc cga tcc ctc gct gta aca gct ctg ggt ggg ctt ccc      48
Met Leu Arg Gly Arg Ser Leu Ala Val Thr Ala Leu Gly Gly Leu Pro
1               5                   10                  15

cag tgg gaa gtc gaa gaa ctt cct gtg gag gag tta ctg ctc ttt gaa      96
Gln Trp Glu Val Glu Glu Leu Pro Val Glu Glu Leu Leu Leu Phe Glu
            20                  25                  30

gtt gct tgg gaa gtg acc aat aaa gtt gga ggc atc tat act gtg att      144
Val Ala Trp Glu Val Thr Asn Lys Val Gly Gly Ile Tyr Thr Val Ile
        35                  40                  45

cag aca aag gcc aaa aca aca gca gat gaa tgg gga gag aac tat ttt      192
Gln Thr Lys Ala Lys Thr Thr Ala Asp Glu Trp Gly Glu Asn Tyr Phe
    50                  55                  60

ctg ata ggt cca tat ttt gag cat aat atg aag act cag gtg gaa cag      240
Leu Ile Gly Pro Tyr Phe Glu His Asn Met Lys Thr Gln Val Glu Gln
65                  70                  75                  80

tgt gaa cct gta aat gat gct gtc aga aga gca gtg gac gca atg aat      288
Cys Glu Pro Val Asn Asp Ala Val Arg Arg Ala Val Asp Ala Met Asn
                85                  90                  95

aag cat ggc tgc cag gtg cat ttt gga aga tgg ctg ata gaa gga agt      336
Lys His Gly Cys Gln Val His Phe Gly Arg Trp Leu Ile Glu Gly Ser

            100                 105                 110

cct tat gtg gta ctt ttt gac ata ggc tat tca gct tgg aat ctg gac 384
Pro Tyr Val Val Leu Phe Asp Ile Gly Tyr Ser Ala Trp Asn Leu Asp
        115                 120                 125

agg tgg aag ggt gac ctc tgg gaa gca tgc agt gtc ggc att cct tat 432
Arg Trp Lys Gly Asp Leu Trp Glu Ala Cys Ser Val Gly Ile Pro Tyr
    130                 135                 140

cat gac cga gaa gcc aat gat atg ctg ata ttt gga tct tta act gcc 480
His Asp Arg Glu Ala Asn Asp Met Leu Ile Phe Gly Ser Leu Thr Ala
145                 150                 155                 160

tgg ttc tta aaa gag gtg aca gat cat gca gat ggt aaa tat gtc gtt 528
Trp Phe Leu Lys Glu Val Thr Asp His Ala Asp Gly Lys Tyr Val Val
                165                 170                 175

gcc caa ttc cat gaa tgg cag gct gga att gga ctg atc ctt tct cga 576
Ala Gln Phe His Glu Trp Gln Ala Gly Ile Gly Leu Ile Leu Ser Arg
            180                 185                 190

gcc agg aaa ctt cct att gcc aca ata ttt aca acc cac gct aca cta 624
Ala Arg Lys Leu Pro Ile Ala Thr Ile Phe Thr Thr His Ala Thr Leu
        195                 200                 205

ctt ggg agg tat ctc tgt gca gca aat att gat ttc tac aac cat ctt 672
Leu Gly Arg Tyr Leu Cys Ala Ala Asn Ile Asp Phe Tyr Asn His Leu
    210                 215                 220

gat aag ttt aac att gac aaa gag gct ggg gaa agg cag att tac cac 720
Asp Lys Phe Asn Ile Asp Lys Glu Ala Gly Glu Arg Gln Ile Tyr His
225                 230                 235                 240

cgg tac tgc atg gag cga gct tcc gtt cat tgc gct cac gtg ttc acc 768
Arg Tyr Cys Met Glu Arg Ala Ser Val His Cys Ala His Val Phe Thr
                245                 250                 255

acg gtt tct gaa ata aca gca ata gaa gct gaa cat atg ctg aag aga 816
Thr Val Ser Glu Ile Thr Ala Ile Glu Ala Glu His Met Leu Lys Arg
            260                 265                 270

aag cct gat gta gtt act cca aac ggc ttg aat gtt aag aaa ttt tca 864
Lys Pro Asp Val Val Thr Pro Asn Gly Leu Asn Val Lys Lys Phe Ser
        275                 280                 285

gca gtg cat gag ttt caa aat cta cat gcc atg tac aag gcc aga atc 912
Ala Val His Glu Phe Gln Asn Leu His Ala Met Tyr Lys Ala Arg Ile
    290                 295                 300

caa gat ttt gtt cga ggt cat ttc tat ggt cat ctc gac ttt gat ctt 960
Gln Asp Phe Val Arg Gly His Phe Tyr Gly His Leu Asp Phe Asp Leu
305                 310                 315                 320

gaa aag act ttg ttc ctt ttc att gct ggg agg tat gag ttt tca aac 1008
Glu Lys Thr Leu Phe Leu Phe Ile Ala Gly Arg Tyr Glu Phe Ser Asn
                325                 330                 335

aaa gga gct gac atc ttc cta gaa tcc tta tcc agg cta aat ttc ctg 1056
Lys Gly Ala Asp Ile Phe Leu Glu Ser Leu Ser Arg Leu Asn Phe Leu
            340                 345                 350

ctg agg atg cat aaa agt gac atc aca gtg gtg gtg ttt ttc att atg 1104
Leu Arg Met His Lys Ser Asp Ile Thr Val Val Val Phe Phe Ile Met
        355                 360                 365

cct gcc aag aca aat aat ttc aac gtg gaa acc ctg aaa gga caa gca 1152
Pro Ala Lys Thr Asn Asn Phe Asn Val Glu Thr Leu Lys Gly Gln Ala
    370                 375                 380

gtg cga aaa cag ctg tgg gat gtt gca cat tct gtg aag gaa aag ttt     1200
Val Arg Lys Gln Leu Trp Asp Val Ala His Ser Val Lys Glu Lys Phe
385                 390                 395                 400

gga aaa aaa ctc tat gat gca tta tta aga gga gaa att cct gac ctg     1248
Gly Lys Lys Leu Tyr Asp Ala Leu Leu Arg Gly Glu Ile Pro Asp Leu
                405                 410                 415

aac gat att tta gat cga gat gat cta aca att atg aaa aga gcc atc     1296
Asn Asp Ile Leu Asp Arg Asp Asp Leu Thr Ile Met Lys Arg Ala Ile
            420                 425                 430

ttt tca act cag cga cag tca ttg ccc cca gtg acc acg cac aac atg     1344
Phe Ser Thr Gln Arg Gln Ser Leu Pro Pro Val Thr Thr His Asn Met
        435                 440                 445

att gat gac tcc acc gac ccc atc ctc agc acc att aga cgg att gga     1392
Ile Asp Asp Ser Thr Asp Pro Ile Leu Ser Thr Ile Arg Arg Ile Gly
    450                 455                 460

ctt ttc aac aac cgc aca gat aga gtc aag gtg att ttg cac cca gag     1440
Leu Phe Asn Asn Arg Thr Asp Arg Val Lys Val Ile Leu His Pro Glu
465                 470                 475                 480

ttt cta tcc tcc acc agt ccc tta cta ccc atg gac tat gaa gag ttt     1488
Phe Leu Ser Ser Thr Ser Pro Leu Leu Pro Met Asp Tyr Glu Glu Phe
                485                 490                 495

gtt aga ggt tgt cat ctt gga gta ttt cca tca tac tat gaa ccc tgg     1536
Val Arg Gly Cys His Leu Gly Val Phe Pro Ser Tyr Tyr Glu Pro Trp
            500                 505                 510

ggt tat act cca gct gaa tgc act gtg atg ggt atc ccc agt gtg acc     1584
Gly Tyr Thr Pro Ala Glu Cys Thr Val Met Gly Ile Pro Ser Val Thr
        515                 520                 525

acg aat ctc tcc ggg ttt ggc tgt ttc atg cag gag cac gtg gct gat     1632
Thr Asn Leu Ser Gly Phe Gly Cys Phe Met Gln Glu His Val Ala Asp
    530                 535                 540

cct act gct tac ggt att tac atc gtt gac agg cgg ttc cgt tct cca     1680
Pro Thr Ala Tyr Gly Ile Tyr Ile Val Asp Arg Arg Phe Arg Ser Pro
545                 550                 555                 560

gat gat tct tgc aat cag ctg act aag ttt ctc tat gga ttt tgc aaa     1728
Asp Asp Ser Cys Asn Gln Leu Thr Lys Phe Leu Tyr Gly Phe Cys Lys
                565                 570                 575

cag tca cgc cgc caa agg att atc cag agg aac aga act gag agg ctc     1776
Gln Ser Arg Arg Gln Arg Ile Ile Gln Arg Asn Arg Thr Glu Arg Leu
            580                 585                 590

tca gat ctt ctg gat tgg aga tac tta ggc aga tat tac cag cat gcc     1824
Ser Asp Leu Leu Asp Trp Arg Tyr Leu Gly Arg Tyr Tyr Gln His Ala
        595                 600                 605

aga cac ctg aca tta agc aga gct ttt cca gat aaa ttc cat gtg gaa     1872
Arg His Leu Thr Leu Ser Arg Ala Phe Pro Asp Lys Phe His Val Glu
    610                 615                 620

cta aca tca cca cca acg aca gaa gga ttt aaa tat ccc agg cct tcc     1920
Leu Thr Ser Pro Pro Thr Thr Glu Gly Phe Lys Tyr Pro Arg Pro Ser
625                 630                 635                 640

gct gta cca cct gct cct tca ggg gct cag gcc tcc gct cct cag agc     1968
Ala Val Pro Pro Ala Pro Ser Gly Ala Gln Ala Ser Ala Pro Gln Ser
                645                 650                 655

gct gat gtg gaa gat gaa gtg gag gat gag aga tac gat gag gaa gag     2016
Ala Asp Val Glu Asp Glu Val Glu Asp Glu Arg Tyr Asp Glu Glu Glu
            660                 665                 670

gag gct gaa agg gat cgg tta aat atc aag tca cca ttt tca ctg agc     2064
Glu Ala Glu Arg Asp Arg Leu Asn Ile Lys Ser Pro Phe Ser Leu Ser
        675                 680                 685

cac gtt cct cat ggg aag aaa aag ctg cat ggt gaa tat aag aac tga     2112
His Val Pro His Gly Lys Lys Lys Leu His Gly Glu Tyr Lys Asn
    690                 695                 700

<210>  14
<211>  703
<212>  PRT
<213>  Artificial

<220>
<223>  human GYS2 AA-5A

<400>  14

Met Leu Arg Gly Arg Ser Leu Ala Val Thr Ala Leu Gly Gly Leu Pro
1               5                   10                  15

Gln Trp Glu Val Glu Glu Leu Pro Val Glu Glu Leu Leu Leu Phe Glu
            20                  25                  30

Val Ala Trp Glu Val Thr Asn Lys Val Gly Gly Ile Tyr Thr Val Ile
        35                  40                  45

Gln Thr Lys Ala Lys Thr Thr Ala Asp Glu Trp Gly Glu Asn Tyr Phe
    50                  55                  60

Leu Ile Gly Pro Tyr Phe Glu His Asn Met Lys Thr Gln Val Glu Gln
65                  70                  75                  80

Cys Glu Pro Val Asn Asp Ala Val Arg Arg Ala Val Asp Ala Met Asn
                85                  90                  95

Lys His Gly Cys Gln Val His Phe Gly Arg Trp Leu Ile Glu Gly Ser
            100                 105                 110

Pro Tyr Val Val Leu Phe Asp Ile Gly Tyr Ser Ala Trp Asn Leu Asp
        115                 120                 125

Arg Trp Lys Gly Asp Leu Trp Glu Ala Cys Ser Val Gly Ile Pro Tyr
    130                 135                 140

His Asp Arg Glu Ala Asn Asp Met Leu Ile Phe Gly Ser Leu Thr Ala
145                 150                 155                 160

Trp Phe Leu Lys Glu Val Thr Asp His Ala Asp Gly Lys Tyr Val Val
                165                 170                 175

Ala Gln Phe His Glu Trp Gln Ala Gly Ile Gly Leu Ile Leu Ser Arg
            180                 185                 190

Ala Arg Lys Leu Pro Ile Ala Thr Ile Phe Thr Thr His Ala Thr Leu
        195                 200                 205

Leu Gly Arg Tyr Leu Cys Ala Ala Asn Ile Asp Phe Tyr Asn His Leu
    210                 215                 220

Asp Lys Phe Asn Ile Asp Lys Glu Ala Gly Glu Arg Gln Ile Tyr His
225                 230                 235                 240

Arg Tyr Cys Met Glu Arg Ala Ser Val His Cys Ala His Val Phe Thr
                245                 250                 255

Thr Val Ser Glu Ile Thr Ala Ile Glu Ala Glu His Met Leu Lys Arg
            260                 265                 270

Lys Pro Asp Val Val Thr Pro Asn Gly Leu Asn Val Lys Lys Phe Ser
        275                 280                 285

Ala Val His Glu Phe Gln Asn Leu His Ala Met Tyr Lys Ala Arg Ile
    290                 295                 300

Gln Asp Phe Val Arg Gly His Phe Tyr Gly His Leu Asp Phe Asp Leu
305                 310                 315                 320

Glu Lys Thr Leu Phe Leu Phe Ile Ala Gly Arg Tyr Glu Phe Ser Asn
                325                 330                 335

Lys Gly Ala Asp Ile Phe Leu Glu Ser Leu Ser Arg Leu Asn Phe Leu
            340                 345                 350

Leu Arg Met His Lys Ser Asp Ile Thr Val Val Val Phe Phe Ile Met
        355                 360                 365

Pro Ala Lys Thr Asn Asn Phe Asn Val Glu Thr Leu Lys Gly Gln Ala
    370                 375                 380

Val Arg Lys Gln Leu Trp Asp Val Ala His Ser Val Lys Glu Lys Phe
385                 390                 395                 400

Gly Lys Lys Leu Tyr Asp Ala Leu Leu Arg Gly Glu Ile Pro Asp Leu
                405                 410                 415

Asn Asp Ile Leu Asp Arg Asp Asp Leu Thr Ile Met Lys Arg Ala Ile
            420                 425                 430

Phe Ser Thr Gln Arg Gln Ser Leu Pro Pro Val Thr Thr His Asn Met
        435                 440                 445

Ile Asp Asp Ser Thr Asp Pro Ile Leu Ser Thr Ile Arg Arg Ile Gly
    450                 455                 460

Leu Phe Asn Asn Arg Thr Asp Arg Val Lys Val Ile Leu His Pro Glu
465                 470                 475                 480

Phe Leu Ser Ser Thr Ser Pro Leu Leu Pro Met Asp Tyr Glu Glu Phe
                485                 490                 495

Val Arg Gly Cys His Leu Gly Val Phe Pro Ser Tyr Tyr Glu Pro Trp
            500                 505                 510

Gly Tyr Thr Pro Ala Glu Cys Thr Val Met Gly Ile Pro Ser Val Thr
        515                 520                 525

Thr Asn Leu Ser Gly Phe Gly Cys Phe Met Gln Glu His Val Ala Asp
    530                 535                 540

Pro Thr Ala Tyr Gly Ile Tyr Ile Val Asp Arg Arg Phe Arg Ser Pro
545                 550                 555                 560

Asp Asp Ser Cys Asn Gln Leu Thr Lys Phe Leu Tyr Gly Phe Cys Lys
                565                 570                 575

Gln Ser Arg Arg Gln Arg Ile Ile Gln Arg Asn Arg Thr Glu Arg Leu
            580                 585                 590

Ser Asp Leu Leu Asp Trp Arg Tyr Leu Gly Arg Tyr Tyr Gln His Ala
        595                 600                 605

Arg His Leu Thr Leu Ser Arg Ala Phe Pro Asp Lys Phe His Val Glu
    610                 615                 620

Leu Thr Ser Pro Pro Thr Thr Glu Gly Phe Lys Tyr Pro Arg Pro Ser
625                 630                 635                 640

Ala Val Pro Pro Ala Pro Ser Gly Ala Gln Ala Ser Ala Pro Gln Ser
                645                 650                 655

Ala Asp Val Glu Asp Glu Val Glu Asp Glu Arg Tyr Asp Glu Glu Glu
            660                 665                 670

Glu Ala Glu Arg Asp Arg Leu Asn Ile Lys Ser Pro Phe Ser Leu Ser
        675                 680                 685

His Val Pro His Gly Lys Lys Lys Leu His Gly Glu Tyr Lys Asn
    690                 695                 700

<210>  15
<211>  61
<212>  DNA
<213>  Artificial

<220>
<223>  GYS2-V-FW-S2,2a

<400>  15
ttttttgaat tccgccacca tgcttcgagg ccgatccctc gctgtaacag ctctgggtgg      60

g                                                                     61


<210>  16
<211>  37
<212>  DNA
<213>  Artificial

<220>
<223>  GYS2-V-RV-S2,2a

<400>  16
ttttttaagc tttcagttct tatattcacc atgcagc                              37